Europäisches Patentamt

European Patent Office

Office européen des brevets

(11) Publication number: **0 435 380 A2**

# EUROPEAN PATENT APPLICATION

(21) Application number: 90203343.0

(51) Int. Cl.⁵: **B01L 3/00**, //C12Q1/68

(22) Date of filing: 14.12.90

(30) Priority: 26.12.89 US 457217

(43) Date of publication of application:
03.07.91 Bulletin 91/27

(84) Designated Contracting States:
BE CH DE DK FR GB IT LI LU NL SE

(71) Applicant: EASTMAN KODAK COMPANY
343 State Street
Rochester, NY 14650-0221(US)

(72) Inventor: Glanville, Thomas William, c/o

EASTMAN KODAK COMP.
Patent Department, 343 State Street
Rochester, New York 14650(US)
Inventor: Bensley, Douglas Steven, c/o
EASTMAN KODAK COMP.
Patent Department, 343 State Street
Rochester, New York 14650(US)

(74) Representative: Phillips, Margaret Dawn et al
Kodak Limited Patent Department Headstone
Drive
Harrow, Middlesex HA1 4TY(GB)

(54) A chemical reaction pack and method of using same.

(57) In techniques for detecting nucleic acid material, there is a risk of contamination during unstoppering of reaction containers and transfer of liquids used in such reactions. This is because aerosols are produced during such unstoppering and transfer, which may contain some amplified nucleic acid material. Described herein are improvements in a disposable chemical reaction pack (10) and method for examining fluids using such a pack, in which liquids can be reacted and examined without the need to unstopper the reaction container and transfer the liquid to a further container for examination. The pack (10) comprises a first chamber (20) into which liquid to be examined is first inserted and sealed. This chamber (20) is connected to a second chamber (24) in which the liquid is examined. A rupturable seal (34) is provided between the first and second chambers (20, 24), which allows liquid to flow from the first chamber (20) into the second chamber (24) once linear pressure has been applied to the liquid in the first chamber (20). Advantageously, an expansion chamber (22) is provided intermediate the first and second chambers to control the velocity of liquid transfer from the first chamber (20) to the second chamber (24).

FIG. 1

## A CHEMICAL REACTION PACK AND METHOD OF USING SAME

The present invention generally relates to a chemical reaction pack and, more particularly, to a disposable chemical reaction pack wherein a test liquid is progressively transferred between adjacent chambers to a detection chamber through which light is passed when an optical reading is taken.

Biological fluid analyzers are capable of performing various testing procedures. During such procedures, a biological fluid, such as serum, plasma, urine, or other fluid, is mixed with a reagent fluid. Chemical reactions between the biological fluid and the reagents, however, require careful processing to avoid contamination.

Polymerase chain reaction (PCR) technology permits nucleic acid material, such as DNA, often extracted from as little as a single cell, to be amplified to hundreds of millions of copies. For example, when a single DNA strand, such as the DNA of the human immunodeficiency virus (HIV, known to cause AIDS) is added to amplifying reagents, hundreds of millions of copies of that DNA can be obtained in a relatively short time.

Such reactions are typically effected in a stoppered plastic containers. To further process or examine the contents of each container, it is reopened, and a test sample is withdrawn or further reagents may be added.

It has been discovered that such a technique is unsatisfactory for convenient and widespread use of PCR technology because aerosols are produced while unstoppering and/or transferring of fluids. Such aerosols contain a few of the amplified nucleic acid materials, e.g. DNA and can be dispersed within the environment. Normally, this is not of great concern. However, only one DNA is needed to contaminate and thereby ruin other amplifying containers.

Although the problem of contamination might be reduced by using highly skilled and trained personnel who painstakingly unstop the containers to minimize the aerosols produced, the need for such intense labor makes the technology impractical for general use. Any design which minimizes operator intervention and lessens the opportunity of contamination during the testing procedure is beneficial.

It is known to provide flexible reaction packs which generally seal the reaction within them. Examples of such packs are disclosed in US-A-4673607. However, the chambers of the pack are constructed so that exteriorly applied pressure breaks the temporary seal to shoot the liquid contents into a common reaction chamber 122. Such a construction causes the ejection of the liquid from its storage chamber at a high pressure and velocity which is essentially uncontrolled. There has been a need, therefore, to provide a reaction vessel which controls the pressure of ejection of the liquid into the subsequent chambers.

It is also known to construct such a flexible reaction pack so that there are three or more chambers in series for liquid transfer, as described, for example, in US-A-3036894. However, each of the chambers is temporarily sealed from further flow downstream thereof. As a result, each chamber only stops the flow into it from an upstream chamber, rather than providing a controlled flow which is slow, albeit continuous, as it proceeds to the desired target chamber further downstream.

It has been a problem to provide a flexible reaction pack with burstable chambers such that flow from a burst chamber proceeds in a manner which is slow, albeit continuous, as it proceeds to the desired target chamber further downstream.

It is therefore an object of the present invention to provide a chemical reaction pack and method for transferring a test liquid such as that used in polymerase chain reaction (PCR) technology in a manner inhibiting contamination. A salient feature of the present invention is that the test liquid is transferred between adjacent chambers in a simplified manner while confining the liquid in a sealed body. This minimizes exposure and the possibility of any of the liquid escaping to the environment.

In accordance with one aspect of the present invention, there is provided a disposable chemical reaction pack comprising:-

a sealed hollow body formed from impermeable pliable sheet material;

a first chamber formed within the body, and arranged to receive liquid from a sample to be reacted;

a second chamber serially connected to the first chamber and being normally open to receive fluid flow from the first chamber; and

a rupturable seal disposed between the first and second chambers for retaining the sample within the first chamber, the rupturable seal being constructed so that it will rupture upon the application of pressure to the liquid in the first chamber;

characterized in that the second chamber includes an expansion chamber located downstream from the seal.

In a preferred form, the rupturable seal forms a wall between the first chamber and the expansion chamber. The seal is readily openable by applying pressure to the first chamber. When a linearly advancing pressure is applied to the first chamber, the test liquid therein is subjected to increasing pressure which stresses the seal. Sufficient pressure on the first chamber results in sufficient internal pressure to open the normally closed fluid communication means between the first chamber and the expansion chamber.

The expansion chamber into which the test liquid is dispersed after the seal has been ruptured, acts to reduce or relieve the pressure of the liquid passing from the first chamber into the second chamber. In the illustrated embodiment, the width of the expansion chamber is greater than the width of the fluid passage joining the two adjacent chambers. Accordingly, as the test liquid passes from the first chamber to the expansion chamber, it quickly expands in an effort to fill the expansion chamber. This expansion effect reduces the pressure of the test liquid.

As linearly advancing pressure is applied to the expansion chamber, the test liquid is freely dispensed therefrom through the normally open fluid passage towards the second chamber. Because there is no need to burst a seal or jointure area, the test liquid flows in a controlled or uniform manner from the expansion chamber to the second chamber. Preferably, the chamber is free of reagents on interior surfaces thereof.

The disposable chemical reaction pack of the present invention is operable under sealed or closed conditions. Thus, in accordance with another aspect of the present invention, there is provided a method of examining a liquid comprising the steps of:

sealing the liquid to be examined in the first chamber of a chemical reaction pack as described above;

applying an external pressure to the first chamber to cause the seal to rupture and to allow at least part of the liquid to continuously transfer out from the first chamber into the second chamber through the expansion chamber; and

examining the liquid in the second chamber;
characterized in that the expansion chamber acts to slow down the velocity of transfer of the liquid from the first chamber to the second chamber.

It is an advantageous feature of the invention that the disposable reaction pack reduces the risk of contaminating the environment since the liquid, albeit transferred between chambers, remains sealed within a closed body. The simplistic design of the present invention minimizes the labor required to be used to operate the system. Moreover, the structure of the closed system of the present invention readily lends itself to automated processing techniques.

The present invention will now be described by way of example only, with reference to the accompanying drawing in which:-

Figure 1 is a top plan view of a chemical reaction pack embodying principles of the present invention;

Figure 2 is a side elevational view of the chemical reaction pack shown in Figure 1;

Figure 3 is a central longitudinal sectioned view taken along line 3-3 of Figure 1;

Figure 4 is a similar central longitudinal sectioned view showing one method of using the reaction pack to carry out a test procedure; and

Figure 5 is a sectioned top plan view taken along line 5-5 of Figure 2.

While the presention is susceptible of embodiment in various forms, there is shown in the drawings and will hereinafter be described a presently preferred embodiment, with the understanding that the present disclosure is to be considered as an exemplification of the invention, and is not intended to limit the invention to the specific embodiment illustrated.

Referring now to the drawings, there is illustrated a disposable chemical reaction pack 10 which is intended to be supported in any orientation, for example a substantially horizontal orientation. The reaction pack 10 comprises a sealed hollow body 12 preferably formed from superimposed and adhered strips 13 and 14 of suitable length and width. Each strip 13, 14 is preferably formed from a fluid impermeable pliable or flexible transparent material, such as polyethylene, polypropylene or a similar plastics material. As used herein, the term "fluid impermeable" is meant to include any material which does not normally allow the rapid or ready passage of either liquids and/or gases therethrough.

The superimposed strips 13, 14 are joined along longitudinal marginal edges 16 and inwardly extending areas 18 by heat fusion or adhesively uniting the strips to each other to provide a series of compartments or chambers surrounded by a perimeter 19 which has a relatively permanent seal, each chamber having a blister-like configuration. The number of compartments formed in body 10 will be dependent upon the number of reaction and detection steps to be performed. The orientation and connection of compartments depends upon the desired protocol.

As illustrated, three compartments or chambers are formed in the body 12 of the present invention. These compartments or chambers comprise a first or burstable chamber 20, a second or expansive chamber 22, and a third or detection chamber 24. To facilitate automated processing of the reaction pack, the chambers 20, 22 and 24 are preferably formed in serial relation to each other. The detection chamber 24 is closed at its outer end by heat fusion or adhesively joining end marginal portions of the strips 13 and 14 to form a closed end 28. Preferably, chamber 24 is expandable in its opposing walls to accommodate the increased volume forced into it.

The burstable chamber 20 is initially open at its outer or inlet end to permit introduction of a test liquid into it. After the introduction of the test liquid into chamber 20, the outer end of the burstable chamber can be closed by heat fusion or adhesively uniting the outer marginal portions of the strips 13 and 14 in the end zone 32.

In the preferred embodiment, the expansion chamber 22 is free of reagent materials while some of the other chambers may contain measured quantities of dried or liquid reagent materials. To facilitate storage, such reagents are preferably in solid form. Moreover, the reagent-containing chambers may be decreased or increased in number depending upon the number of reactions in a test procedure or the number of test procedures desired to be performed.

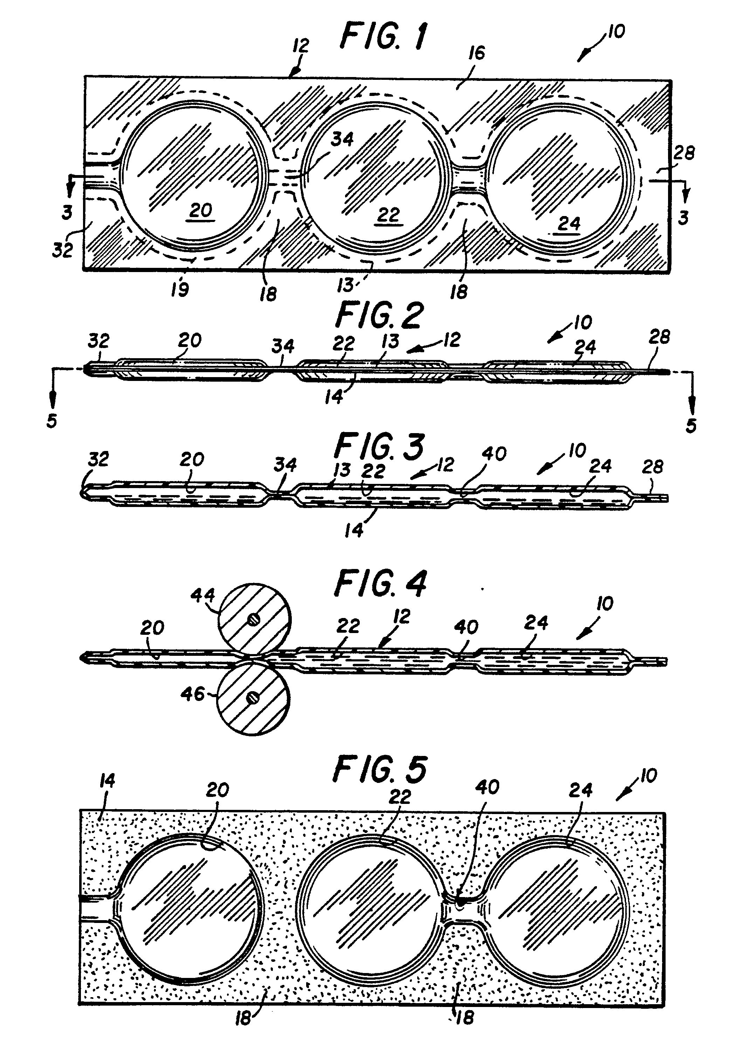

The burstable chamber 20 and expansive chamber 22 are normally closed relative to one another by temporary separating or sealing means which open or rupture under application of internal pressure in contrast to the perimeter seal at portions 19 which is permanent. As illustrated, the separating or sealing means may comprise a narrow transverse heat-sealed or adhesive jointure 34, as shown in Figure 1, which forms a rupturable seal or wall between bursting chamber 20 and the expansion chamber 22. The jointure 34 is preferably designed to burst or open wide rather than narrow. Due to its relatively small area, the jointure 34 readily yields to applied external pressure thereby opening communication between the burstable chamber 20 and expansion chamber 22 during operative manipulation of the body 12 as hereinafter set forth.

In a preferred form and as illustrated, chamber 22 is considerably wider than the jointure area 34 between chambers 20 and 22. The configuration and position of the expansion chamber 22 relative to the jointure area 34 is designed to relieve pressure on the pressurized test liquid ejected from the burstable chamber 20 so that the flow of liquid into the detection chamber can be controlled. Although chamber 22 is shown as being about equal in volume to that of chamber 20, it need not be; preferably the volume of chamber 22 is at least 50% that of chamber 20. Larger volumes are required if considerable air is also present in chamber 20.

As illustrated in Figures 3 and 5, downstream of the first or burstable chamber 20, body 12 defines a normally open fluid passage 40 leading from the second or expansion chamber 22. In the illustrated embodiment, the fluid passage 40 leads to and readily allows passage of fluid between the expansion chamber 22 and the detection chamber 24.

When it is desired for a test liquid to react with one or more reagents, appropriate reagents in predetermined appropriate amounts are deposited in successive chambers during fabrication of the reaction pack 10 of the present invention. As an example, when a liquid is to react with two reagents, a measured amount of a first reagent can be enclosed within the burstable chamber 20 and a measured amount of a second reagent can be deposited within the third chamber 24. Thereafter, a liquid is introduced into the burstable chamber 20 which is then closed to permit a predetermined incubation or reaction period to begin. Optionally, reagents can be preapplied to chamber 22.

When the predetermined incubation or reaction period is complete, pressure is linearly applied to the burstable or first chamber 20. Such pressure may be applied either manually or mechanically. If the pressure is applied mechanically, the pressure is preferably applied by linearly advancing the hollow body 12 through compression means at a predetermined linear rate. As illustrated in Figure 4, such compression means can include a pair of rollers 44 and 46 arranged on opposite sides of the body 12 in between which the body linearly passes.

As body 12 passes between the rollers 44 and 46, the test liquid in the burstable chamber 20 is progressively squeezed between the rollers 44, 46 and is forced toward the rupturable seal 34, thereby exerting internal pressure against the normally closed seal. Sufficient internal pressure acting against the seal 34 will rupture the seal between the burstable chamber 20 and the expansion chamber 22.

Rupture of the seal will allow transfer of the test liquid from the burstable chamber 20 into the expansion chamber 22. The shape and position of the expansion chamber 22 into which the test liquid is dispersed inherently provides an expansion effect which will slow down the rush of liquid which is ejected from the burstable chamber 20. Because the expansion chamber is configured with a larger area than is that provided for the passage opening thereto, the expansion chamber 22 receives the liquid from the burstable chamber 20 and the flow rate from the expansion chamber 22 is slower and more controlled.

The sealed body 12 continues linear movement through the rollers 44, 46 which impart the pressure onto the body to advance or transfer the liquid from the expansion chamber 22 through the open passage 40 and into the chamber 24. Once in the chamber 24, the liquid intermixes with and reacts with the reagent. As will be understood, the test liquid is permitted to freely flow between the second and third chambers 22 and 24 respectively through the normally open fluid passage 40. Accordingly, the test fluid is transferred or metered from the second chamber 22 at a uniform rate determined by the rate of linear advancement of body 12 between rollers 44, 46.

If the reactions occurring in the chambers require thermal treatment, a portion or the entirety of the sealed body 12 may be subjected to a temperature cycling process extending through a temperature range of from about 30°C to about 95°C or any other temperature range in any of several methods of treatment.

A salient feature of the present invention is that the simplicity of the present invention readily allows transfer of the liquid to be readily effected with minimum operator involvement and furthermore, in a manner which lends itself to automated processing.

The reaction pack according to the present invention is not limited to use with PCR chemistries, but is also useful with other chemistries, for example chemistries for the LCR method of amplifying DNA.

**Claims**

1. A disposable chemical reaction pack (10) comprising:-
   a sealed hollow body (12) formed from impermeable pliable sheet material;
   a first chamber (20) formed within the body (12), and arranged to receive liquid from a sample to be reacted;
   a second chamber (24) serially connected to the first chamber (20) and being normally open to receive fluid flow from the first chamber (20); and
   a rupturable seal (34) disposed between the first and second chambers (20, 24) for retaining the sample within the first chamber (20), the rupturable seal (34) being constructed so that it will rupture upon the application of pressure to the liquid in the first chamber (20);
   characterized in that the second chamber (24) includes an expansion chamber (22) located downstream from the seal (34).

2. A pack according to claim 1, wherein the rupturable seal (34) forms a wall between the first chamber (20) and the expansion chamber (22).

3. A pack according to claim 1 or 2, wherein the chambers (20, 22, 24) are disposed within the hollow body (12) such that liquid contained within the first chamber (20) can be progressively transferred from one chamber to another by applying external pressure linearly to the hollow body (12).

4. A pack according to any one of the preceding claims, wherein some of the chambers (20, 22, 24) have reagents therewithin.

5. A method of examining a liquid comprising the steps of:
   sealing the liquid to be examined in the first chamber (20) of a chemical reaction pack (10) according to any one of the preceding claims;
   applying an external pressure to the first chamber (20) to cause the seal (34) to rupture and to allow at least part of the liquid to continuously transfer out from the first chamber (20) into the second chamber (24) through the expansion chamber (22); and
   examining the liquid in the second chamber (24);
   characterized in that the expansion chamber (22) acts to slow down the velocity of transfer of the liquid from the first chamber (20) to the second chamber (24).

*FIG. 1*

*FIG. 2*

*FIG. 3*

*FIG. 4*

*FIG. 5*